# FASCICULE DE BREVET EUROPEEN

(11) **EP 3 478 225 B1**
(45) Date de publication et mention de la délivrance du brevet: **12.03.2025**
(21) Numéro de dépôt: 17732963.8
(22) Date de dépôt: 30.06.2017
(51) Int. Cl.: A61F 2/46, A61B 17/88, A61L 31/00

(54) **SUBSTITUT OSSEUX ET SYSTEME D'INJECTION AUTONOME**
KNOCHENERSATZ UND UNABHÄNGIGES EINSPRITZSYSTEM
BONE SUBSTITUTE AND INDEPENDENT INJECTION SYSTEM

(30) Priorité: 30.06.2016 FR 1656231
(43) Date de publication de la demande: 08.05.2019
(73) Titulaire: Teknimed, 65500 Vic-en-Bigorre (FR)
(72) Inventeur: IEHL, Julien, 31170 TOURNEFEUILLE (FR); HALBIN, Gautier, 31000 TOULOUSE (FR); SENDER, Cyril, 31500 TOULOUSE (FR); SAHRAOUI, Nouredine, 31400 TOULOUSE (FR)
(74) Mandataire: Plasseraud IP
(86) Numéro de dépôt international: PCT/EP2017/066346
(87) Numéro de publication internationale: WO 2018/002335

(56) Documents cités:
- WO-A2-2007/028120
- US-A1- 2005 245 938
- US-A1- 2007 299 426
- US-A1- 2008 249 530
- US-A1- 2010 030 220
- US-A1- 2010 211 058

## Description

La présente invention concerne le domaine des substituts osseux biorésorbables ou non, destinés à l'implantation chez l'homme visant à reconstituer le stock osseux en comblant une perte de substance.

L'invention concerne plus particulièrement le domaine du comblement osseux sous contrainte mécanique.

L'ostéoporose est un problème médical important, et de plus en plus un problème économique important. Plus du tiers des femmes et environ un homme sur sept de 50 ans présenteront une fois dans leur vie une fracture secondaire à leur ostéoporose.

La fracture du corps vertébral est la plus fréquente. La première vertébroplastie transcutanée a été effectuée en 1984. Galibert et al. ont traité un angiome vertébral cervical par ponction transcutanée et remplissage du corps vertébral par un ciment polyméthylmétacrylate (PMMA). Depuis plusieurs années, la vertébroplastie est de plus en plus acceptée dans le traitement des fractures vertébrales ostéoporotiques.

La vertébroplastie consiste à injecter un ciment, par exemple acrylique, par voie percutanée dans les corps vertébraux sous contrôle fluoroscopique et/ou tomographique. La méthode de la vertébroplastie a été perfectionnée, dans le but de redonner au corps vertébral généralement cunéiformisé sa forme originelle. La kyphoplastie consiste à dilater (ie réduire) le corps vertébral au moyen d'un ballonnet gonflable avant d'injecter du ciment.

Ces techniques permettent d'atténuer les douleurs, de limiter les analgésiques et d'abréger l'hospitalisation. Dans les 72 heures après une vertébroplastie, 85 à 90% des patients ayant des fractures vertébrales ostéoporotiques bénéficient d'une atténuation remarquable, voir totale, de leurs douleurs.

Les résines acryliques présentent une résistance mécanique élevée de l'ordre de 80 à 90 MPa en résistance à la compression, et sont utilisées dans ces techniques de comblement osseux sous contrainte. Or, le ciment PMMA présente plusieurs inconvénients. Il est reconnu que le durcissement in vivo des ciments à base de PMMA génère des radicaux libres. Par ailleurs, la réaction de polymérisation de ces matériaux lors de leur préparation extemporanée au bloc opératoire est exothermique et la chaleur dégagée pendant le durcissement est élevée, pouvant dépasser les 90°C, et peut endommager les tissus environnants. Les substituts osseux résorbables et biocompatibles tels que les céramiques phosphocalciques, les bioverres et autres dispositifs médicaux implantables disponibles sous forme de blocs ou de granules présentent une résistance mécanique insuffisante. Les ciments phosphocalciques, malgré leur durcissement in situ et une résistance mécanique de 4 à 20 MPa pour les plus résistants, ne répondent pas non plus au cahier des charges des techniques de comblement osseux sous contrainte. En outre, le durcissement de ces polymères est irréversible et une fois celui-ci initié, le chirurgien ne dispose que d'une fenêtre de temps réduite pour l'injection. Aussi, si pour une raison ou un autre, le chirurgien doit interrompre l'injection et que la cavité à combler n'est pas remplie, il doit tout recommencer et jeter l'équipement d'injection contenant le polymère durci.

On a ainsi vu se développer des substituts osseux associant un agent de type céramique phosphocalcique ou de type os avec un ou des polymères biorésorbables.

Par exemple EP2395949 décrit un matériau substitut osseux qui est fluide ou pâteux lorsqu'il est chauffé à une température de 70 à 95°C et ainsi extrudable et injectable en un site d'implantation. Une fois refroidi, le matériau se solidifie et se résorbe, laissant place à du tissu osseux. Il est en outre indiqué que l'invention décrite par EP2395949 repose sur le principe de chauffer un substitut osseux qui devient fluide à une température élevée via un dispositif utilisé pour administrer ledit matériau chauffé.

De très nombreuses combinaisons sont décrites telles que des composites os/polymère, minéral/polymère, céramique/polymère. Le polymère peut être un polycaprolactone, un poly(lactide-co-glycolide) ou encore un polyuréthane. Des exemples de matériaux substituts osseux composites sont décrits en référence aux documents US2005/0008672, WO04/53112, WO2007/084725, US2008/0069852 et US2007/0191963. Ces documents décrivent des ostéoimplants comprenant une association de polymères d'origine et de nature chimique variées associés à un dérivé osseux, un matériau inorganique, un matériau substitut osseux ou d'une combinaison de ceux-ci.

WO2003/059409 décrit un implant comprenant un sulfate de calcium anhydre additionné d'un polymère bioabsorbable et de phosphate calcique. Le polymère bioabsorbable peut être un polymère ou copolymère de l'acide lactique, de l'acide glycolique, de l'acide hydroxybutyrique, de l'acide hydroxyvalérique, de polydioxane, de polycaprolactone, d'oxyde de poly ethylène ou polyéthylène térephtatate. Cependant, ce document ne divulgue pas d'implant étant apte à être utilisé par chauffage à une température de l'ordre de 60° permettant sa fusion, ou à tout le moins son ramollissment permettant son extrusion, et son administration en un point osseux particulier.

US2008/0003255 décrit des matériaux thermoplastiques pour la réparation osseuse. Y sont décrits les polyesters aliphatiques et en particulier le polycaprolactone comme matériau approprié. Il est indiqué que la polycaprolactone de poids moléculaire supérieur à 100 000 présente une température de fusion située aux environs de 60°C qui lui permet d'être injectée. Il est aussi indiqué que ce matériau peut être chauffé et injecté à l'aide de pistolet chauffant équipé de résistance et de boucle de régulation thermique.

US 8562619 décrit une méthode, un matériau et un dispositif d'injection dudit matériau pour le comblement et/ou la réparation osseuse. La méthode explicitée consiste à chauffer une composition de ciment osseux afin de rendre cette composition fluide, de l'injecter dans la zone à combler pour qu'elle s'y solidifie. Il est indiqué que la température d'injection est inférieure à celle de la fonte initiale afin d'éviter la nécrose mais permet cependant de maintenir la fluidité. Il est indiqué que la composition de ciment osseux comprend un polymère biocompatible thermoplastique dont la température de fusion est comprise entre 70 et 200°C en complément de phosphate de calcium. Le polymère biocompatible thermoplastique décrit peut être choisi parmi : poly(lactic-co-glycolic acide, polylactic acide, poly glycolic acide, polyhydroxybutyrate, polyalkylene succinate, polybutylene diglycolate, polycaprolactone et leurs combinaisons. Les seuls exemples divulgués concernent le poly-lactic-glycolic acide et les températures de fusion des mélanges testés se situent dans une gamme supérieure à 100°C. Les compositions décrites dans ce document nécessitent la mise en œuvre de températures très élevées afin d'assurer leur fluidité.

WO2010/0030220 décrit un système comprenant plusieurs sections dont une chambre constituant un réservoir de ciment destiné à la préparation de celui-ci, un injecteur sous la forme d'une aiguille et une chambre d'activation disposée entre la chambre réservoir et l'injecteur. La chambre d'activation est connectée à une source d'énergie alimentant les moyens de chauffage compris dans la section correspondant à la chambre d'activation. Le ciment est composé de deux composants et d'un catalyseur. La viscosité du mélange est très variable et ce document enseigne un chauffage de la composition via la chambre d'activation et décrit qu'une viscosité très important mais stable peut être obtenue via une boucle de régulation thermique du mélange en cours de polymérisation. Un tel système est complexe et couteux à fabriquer.

US 2010/030220, WO 2007/028120, US 2008/249530, US 2010/0211058 décrivent également des systèmes d'injection de ciment osseux.

En particulier, il convient de noter que tous les documents cités ci-avant nécessitent l'emploi de dispositifs de chauffage et de délivrance de la composition complexes et onéreux.

Typiquement il s'agit de dispositifs de type pistolet doté d'un réservoir et d'une canule associés à un système de chauffage et de thermorégulation intégré qui délivre une chaleur suffisante pour rendre et maintenir fluide le matériau substitut osseux.

Dans de tels dispositifs la quantité de chaleur est régulée soit directement par l'usager ou via un système de feedback électronique afin de maintenir le matériau fluide. De tels dispositifs de délivrance comprennent en général une poignée, un boitier et une chambre pour le matériau à délivrer. Ces dispositifs ressemblent à un pistolet doté d'un réservoir destiné à recevoir le matériau de type substitut osseux tel que décrit supra, d'une canule et de moyens de chauffage et de thermostatation (intégrés ou déportés) du matériau au niveau du réservoir et du début de la canule afin de maintenir le matériau sous forme fluide avant son injection dans une zone d'implantation.

Ces dispositifs sont complexes tant dans leur fabrication, leur utilisation que leur maintenance. Ils sont aussi onéreux et lourds à manipuler par un chirurgien. En outre, pour assurer la thermostatation, une connexion électrique filaire ou une batterie est nécessaire, ce qui complexifie encore l'usage et/ou crée un risque électrique pour le patient. Enfin, leur utilisation implique des étapes de manipulation de matériau de type substitut osseux afin de « charger » la chambre de chauffage et d'extrusion du dispositif. De tels équipements ne sont utilisables qu'avec des préparations extemporanées de matériaux polymères durcissant car leur préparation, leur stockage et leur chauffage sont réalisés dans des enceintes ou sections différentes. En outre, après chaque utilisation, un tel dispositif de type pistolet est jeté. A chaque utilisation, le dispositif de type pistolet stérile doit être assemblé et chargé avec une dose de matériau de comblement stérile préparée, mis en chauffage et thermostaté. Toutes ces manipulations doivent être réalisées en maintenant la stérilité et l'asepsie, ce qui pour des équipements lourds et complexes est soit très compliqué soit très onéreux. Comme indiqué plus avant, une fois utilisé le dispositif est jeté. En outre, si en cours d'utilisation, le chirurgien doit interrompre le geste opératoire, l'injection ne peut pas être reprise car la polymérisation est irréversible.

Il existe donc un besoin en de nouveaux système de matériaux sous de nouveaux formats/sytèmes de délivrance associés à de nouveaux dispositifs pour leur manipulation et leur injection dans le cadre de techniques de comblement osseux sous contrainte mécanique élevée permettant de résoudre les problèmes précités en fournissant simplicité, confort, commodité d'usage pour le chirurgien tout en assurant la sécurité pour le patient.

C'est ainsi un premier objet de la présente invention que de fournir une composition pour comblement osseux conditionnée sous la forme de dose à usage unique, telle qu'un bâtonnet ou des granulés, comprenant, ou consistant en, au moins un matériau polymère thermoplastique résorbable ou non résorbable, caractérisée en ce que ladite composition présente une viscosité comprise entre 40 et 4000 Pa.s, en particulier entre 50 et 3000 Pa.s, plus particulièrement encore entre 80 et 2500 Pa.s, plus particulièrement encore entre 150 et 2000 Pa.s, entre 200 et 1500 Pa.s et à une température de 50°C environ et un module de Young compris entre 0.5 et 4 GPa, particulièrement entre 1 et 4 GPa, entre 1.5 et 3.5 GPa, plus particulièrement encore entre 2 et 3 GPa, et ce à 37°C environ.

L'expression dose à usage unique s'entend dans le contexte de la présente invention en ce que la quantité de composition est adaptée pour une intervention chirurgicale sur un patient pour au moins un comblement osseux ou tout type d'intervention impliquant un complément de matière à un endroit ou de l'os fait défaut. Cette dose à usage unique s'entend aussi qu'elle est continue et sous forme à usage unique et adaptée à l'utilisation dans un dispositif selon l'invention tel que décrit plus loin. Elle peut se présenter sous la forme de bâtonnet ou de granulés du matériau polymère.

Le matériau polymère peut être résorbable ou non résorbable selon les indications envisagées et est thermoplastique. Le matériau polymère est déjà polymérisé et il n'est plus nécessaire de procéder à une préparation extemporanée de réactifs monomériques et de catalyseur.

Le matériau polymère résorbable ou non résorbable sera choisi en fonction de se température de fusion qui sera comprise entre environ 60°C et 90°C.

Cette notion de température de fusion ne constitue par une barrière en tant que telle. Des matériaux ayant une température de fusion supérieure à 90°C mais tout en étant malléables à 60°c peuvent convenir. En effet les inventeurs ont constaté que même si le polymère ou la composition le comprenant est chauffé à une température proche tout en étant inférieure à la température de fusion, le polymère ou la composition le comprenant peut être poussé hors de la seringue et extrudé à travers la filière et l'aiguille d'injection.

En effet, à l'approche de la température de fusion, les « liaisons faibles » entre les chaînes des polymères deviennent fragiles sous l'action de l'agitation thermique, le polymère devient souple et capable de se déformer élastiquement ou plastiquement sans rupture. Cette propriété justifie en partie l'utilité de la plupart des polymères selon l'invention.

Il n'est ainsi pas nécessaire de se situer à la température de fusion (ou au-delà de celle-ci) qui peut en effet être supérieure à la température admissible et compatible avec les tissus du patient.

De manière avantageuse le matériau polymère résorbable ou non résorbable est un polymère thermoplastique.

Le matériau polymère est choisi tel que la composition présente ainsi une viscosité comprise entre 40 et 4000 Pa.s, plus particulièrement 50 et 3000 Pa.s, plus particulièrement encore entre 80 et 2500 Pa.s, plus particulièrement encore entre 150 et 2000 Pa.s, entre 200 et 1500 Pa.s et ce à une température d'environ 50°C.

Cette caractéristique traduit le fait que la composition est fluide ou malléable à cette température et peut ainsi être aisément extrudée à travers une canule et injectée en un point osseux chez un patient et ce sans risque de brulure ou de nécrose des tissus de ce patient.

En outre le matériau polymère de la composition est tel que ladite composition présente après refroidissement, et plus particulièrement à une température d'environ 37°C, une dureté telle que le module d'Young de ladite composition est compris entre 0.5 et 4 GPa, particulièrement entre 1 et 4 GPa, entre 1.5 et 3.5 GPa, plus particulièrement encore entre 2 et 3 GPa, et ce à 37°C environ.

L'expression « fluide ou malléable » s'entend ainsi d'une composition qui est rendue coulante ou molle, dont la viscosité est comprise entre les valeurs indiquées ci-avant, c'est à dire entre 40 et 4000 Pa.s, à une température d'environ 50°C et est par conséquent extrudable et injectable. La composition rendue « fluide ou malléable» par chauffage peut ainsi passer à travers une canule de diamètre compris entre 8 et 15 gauges. Le chauffage de la composition à une température proche ou supérieur à la température de fusion du polymère permet la fluidisation, ou à tout le moins son ramollissement, de ladite composition permettant ainsi sa progression à travers la cavité d'une canule par application d'une pression afin d'assurer son injection.

Pour certain polymères dont la température de fusion est supérieure à 50°C ou 60°C, par exemple de l'ordre de 80, 90 ou 100°C ; il a été remarqué qu'on pouvait les chauffer aux environs de 100 ou 120°C afin d'atteindre ou dépasser le point de fusion puis les refroidir lentement jusqu'aux environs de 50 ou 60°C et qu'ils conservaient une malléabilité permettant leur extrusion et leur injection de la seringue et à travers l'aiguille sans effort démesuré.

Le module de Young de la composition après refroidissement, en particulier à 37°C, est tel qu'il est avantageusement compris entre 2 et 3 GPa.

Le module de Young ou module d'élasticité (longitudinale) ou encore module de traction est la constante qui relie la contrainte de traction (ou de compression) et le début de la déformation d'un matériau.

La loi d'élasticité est la loi de Hooke, c'est-à-dire σ = E ε où :
- σ: est la contrainte (en unité de pression) ;
- E: est le module de Young (en unité de pression) ;
- ε: est l'allongement relatif, ou déformation (adimensionnel).

Le module de Young est la contrainte mécanique qui engendrerait un allongement de 100 % de la longueur initiale d'un matériau (il doublerait donc de longueur), si l'on pouvait l'appliquer réellement : dans les faits, le matériau se déforme de façon permanente, ou se rompt, bien avant que cette valeur ne soit atteinte. Le module de Young est la pente initiale de la courbe de déformation-contrainte.

Un matériau dont le module de Young est très élevé est dit rigide. Le module d'Young peut être mesuré selon la méthode DIN EN ISO 527-2 par exemple.

Une telle rigidité permet d'assurer une résistance mécanique élevée de la composition après son implantation dans une cavité osseuse et de constituer ainsi un ostéoimplant durable ou résorbable selon la nature et la qualité des composants de la composition.

La sélection du matériau polymère thermoplastique, résorbable ou non résorbable, relève de pratiques et mesures classiques pour un homme du métier sur la base des caractéristiques physico-chimiques (par exemple la température de fusion) et rhéologiques qui sont soient connues soient mesurables selon des techniques de laboratoires à sa portée.

Par « ostéoimplant » on entend ici tout matériau servant à aider ou à augmenter une formation osseuse défectueuse. Les ostéoimplants sont souvent appliqués pour combler et complémenter un défaut osseux résultant d'une blessure, d'une malformation, d'une déformation, d'un défaut ou encore d'une chirurgie par exemple. Un ostéoimplant peut être utilisé dans une variété de chirurgies, qu'il s'agisse de chirurgie orthopédique ou neurochirurgie par exemple.

Le matériau polymère de la composition selon l'invention pourra être un polymère résorbable thermoplastique et être choisi dans le groupe constitué par l'acide poly(lactique-co-glycolique), l'acide polylactique (PLA), l'acide polyglycolic (PLGA), le poly(caprolactone) (PCL), le polyhydroxybutyrate, le polyglycerolsebacate, le poly(dioxanone) utilisés seuls ou en mélange. De préférence il sera choisi parmi l'acide poly(lactique-co-glycolique), l'acide polylactique (PLA), l'acide polyglycolic (PLGA), le poly (caprolactone) (PCL), utilisés seuls ou en mélange.

Le polymère thermoplastique résorbable pourra être un mélange physique ou un mélange chimique des composés cités ci-avant. Ainsi par mélange chimique on pourra entendre des copolymères PCL-PLA ou PCL-PGA ou encore PCL-PLGA. Par mélange physique on entend un mélange comprenant des polymères, par exemple PCL PLA mais dont les unités monomériques ne sont pas liées par liaisons covalentes comme dans la cas de copolymères. On pourra ainsi avoir des mélanges physiques PCL-PLA, PCL-PGA, PCL-PLGA en des ratios variables. L'intérêt d'intégrer du PCL dans un mélange contenant du PLA ou du PGA ou du PLGA est d'abaisser le point de fusion du PLA ou PGA ou PLGA et de conserver la rigidité une fois le mélange refroidi.

Le polymère de la composition selon l'invention pourra être un polymère non résorbable thermoplastique et être choisi dans le groupe constitué par les polymères et copolymères acrylates et méthacrylate, polyuréthane, polyéthylène, utilisé seul ou en mélange, de préférence parmi les polymères et copolymères acrylates et méthacrylate.

Parmi les polymères et copolymères acrylates et méthacrylate on peut choisir les polyméthacrylate d'alkyle en C6-C18 ou un mélange de ceux-ci, par exemple le polyméthacrylate de stéaryl, qui présentent des températures de fusion plus basses que le polyméthacrylate de méthyle par exemple. On peut ainsi préparer des mélanges physiques de polyméthacrylates d'alkyle de C1 à C18 dans lesquels les polymères à chaine latérale plus ou moins longue sont en quantité variable. De tels mélanges physiques permettent d'obtenir des mélanges de polymères thermoplastiques présentant une température de fusion proche de 100°C tout en conservant la rigidité souhaitée.

Dans un mode de réalisation particulier le polymère est un polymère résorbable thermoplastique et il s'agit de polycaprolactone seul ou mélangé à un autre polymère résorbable thermoplastique.

Dans un mode de réalisation particulier le polymère est une polycaprolactone de poids moléculaire compris entre 10000 et 100000, particulièrement entre 30000 et 60000, plus particulièrement entre 30000 et 50000, plus particulièrement aux environs de 40000.

Le poids moléculaire du polycaprolactone détermine la température de fusion de ce polymère et donc de la composition. Plus le poids moléculaire est faible, plus la température de fusion est basse. Cependant, ce poids moléculaire détermine aussi la rigidité du matériau à une température de l'ordre de 37°C, c'est-à-dire la température corporelle. Ainsi un poids moléculaire faible entraine une friabilité du matériau durci, ce qui n'est pas acceptable. D'autre part, la viscosité du matériau chauffé au-dessus de sa température de fusion est en grande partie déterminée par son poids moléculaire. Aussi la viscosité est proportionnelle au poids moléculaire.

On pourra donc choisir un polymère dont la viscosité à l'état fondu ou à l'état malléable permet d'une part une extrusion et une injection à une température de l'ordre de 50°C de la composition et ce sans effort démesuré tout en présentant une température compatible avec les tissus biologiques du patient.

Enfin, la résistance mécanique et la rigidité de la composition une fois en place et refroidie à température corporelle doit être suffisamment élevée pour jouer son rôle de matériau de comblement et résister aux contraintes mécaniques.

C'est ainsi que le polymère pourra être choisi par l'homme du métier par des essais de routine consistant à mesurer la température de fusion et la viscosité associée et de retenir les polymères permettant d'obtenir une composition dont la viscosité est telle qu'indiquée plus avant, à la température de 50°C environ.

Une seconde grille de sélection du polymère convenable consiste à mesurer le module d'Young de la composition comprenant, ou consistant en, le polymère et à retenir celui qui permet d'obtenir un module d'Young de la composition comprise entre 0.5 et 4 GPa, particulièrement entre 1 et 4 GPa, entre 1.5 et 3.5 GPa, plus particulièrement encore entre 2 et 3 GPa, et ce à 37°C environ.

Dans un autre mode de réalisation, la composition sous forme de dose à usage unique, telle qu'un bâtonnet ou des granulés, selon l'invention comprend en outre un matériau inorganique, plus particulièrement un biomatériau inorganique, plus particulièrement encore un biomatériau phosphocalcique.

Aussi, le ratio massique polymère/matériau inorganique de la composition selon l'invention peut être comprise en 100/0 et 40/60 par exemple, plus particulièrement entre 90/10 et 50/50, plus particulièrement encore entre 80/20 et 70/30.

Ce ratio influence aussi la viscosité de la composition au moment de l'injection ainsi que la résistance de la composition solidifiée, c'est-à-dire l'ostéoimplant, une fois disposé dans la cavité qui est comblée. Le ratio sera déterminé en conjonction avec la nature du polymère et de la viscosité dudit polymère et de sa dureté afin que la viscosité de la composition à 50°C soit comprise entre 40 Pa.s et 4000 Pa.s et que le module d'Young de la composition à 37°C environ soit compris entre 0.5 et 4 GPa, particulièrement entre 1 et 4 GPa, entre 1.5 et 3.5 GPa, plus particulièrement encore entre 2 et 3 GPa.

La dose à usage unique de la composition selon l'invention est dimensionnée pour correspondre à un ou plusieurs comblements osseux et est compris entre 2 et 50 ml, préférentiellement entre 4 et 12 ml.

De manière avantageuse la dose à usage unique se présente sous la forme de bâtonnet de section cylindrique dont les dimensions peuvent être de l'ordre de 4 à 10 cm de long et 1 à 3 cm de diamètre. Un tel format permet l'utilisation d'une telle dose dans un dispositif tel qu'exposé plus loin dans la description.

Dans un autre mode, la dose à usage unique peut se présenter sous la forme de granulés et la quantité de ces granulés présents dans la seringue correspond à la dose de comblément.

Selon un mode de réalisation, le matériau inorganique de type biomatériau phosphocalcique est une biocéramique phosphocalcique, particulièrement une biocéramique phosphocalcique strontique.

Le matériau phosphocalcique, tel qu'une biocéramique phosphocalcique, présente l'avantage d'être biorésorbable.

De préférence, ladite biocéramique comprend ou consiste en un ou plusieurs composés phosphocalciques frittés choisis dans le groupe consistant en hydroxyapatite (HA), alpha- et beta-tricalcium phosphate (α-TCP, β-TCP) et matériau phosphocalcique biphasé (BCP) ou un de leurs mélanges.

De manière particulièrement préférée, ladite biocéramique comprend un matériau phosphocalcique biphasé, comprenant ou consistant en environ 80% HA et environ 20% TCP ou environ 20% HA et environ 80% β-TCP.

Dans un mode de réalisation le matériau inorganique de base est un biomatériau phosphocalcique résorbable. De manière particulière, le biomatériau phosphocalcique est complémenté par une substitution de un ou plusieurs atomes de calcium par des atomes de strontium. L'apport d'atome de strontium permet d'augmenter proportionnellement la radio-opacité du biomatériau final.

Le composé inorganique de type biomatériau phosphocalcique peut être choisi dans le groupe comprenant le phosphate de calcium amorphe (ACP), Caₓ(PO₄)y.H₂O; le phosphate monocalcique monohydraté (MCPH), CaH₄(PO₄)₂.H₂O ; le phosphate dicalcique dihydraté (DCPD), CaHPO₄.2H₂O; le phosphate dicalcique anhydre (DCPA), CaHPO₄ ; l'apatite précipitée ou déficiente en calcium (CDA), (Ca,Na)₁₀(PO₄,HPO₄)₆(OH)₂ ; l'alpha- ou beta- tricalcium phosphate (α-TCP, β-TCP), Ca₃(PO₄)₂ ; et le tétracalcium phosphate (TTCP), Ca₄P₂O₉.

Plus précisément, la composition selon l'invention sous forme de dose à usage unique se présente sous forme de bâtonnet, ou de granulés, et comprend en poids 40 à 100 % d'un polymère de polycaprolactone de poids moléculaire compris entre 10000 et 100000, plus particulièrement entre 30000 et 60000 , 0 à 60 % d'un biomatériau phosphocalcique, en particulier un biomatériau phosphocalcique comprenant de l'hydroxyapatite et/ou du phosphate tricalcique.

Avantageusement, le polymère de polycaprolactone est résorbable.

Avantageusement, le ratio en poids hydroxyapatite/phosphate tricalcique dans le biomatériau phosphocalcique est compris entre 80/20 et 20/80, plus avantageusement encore compris entre 65/35 et 35/65.

Selon un mode de réalisation particulièrement avantageux de l'invention, le biomatériau phosphocalcique selon l'invention comprend en poids 65% d'hydroxyapatite et 35% de phosphate tricalcique strontique.

Le matériau inorganique de la composition selon l'invention peut aussi être choisi dans le groupe constitué par les bioverres, les silicates, en particulier les aluminosilicates, les sulfates calciques, les sulfates de barium.

La composition selon l'invention peut comprendre des composés et molécules organiques telles que des polysaccharides, des protéines ou des peptides, des lipides par exemple.

Les compositions selon l'invention présentent une grande utilité pour de nouvelles techniques chirurgicales de plus en plus pratiquées, comme la vertébroplastie ou la kyphoplastie ou l'ostéoplastie plus généralement par exemple.

Ces opérations nécessitent des matériaux de nature radio opaque (de façon permanente ou non suivant les cas). Elles sont en effet réalisées sur des vertèbres plus ou moins fracturées et la fuite de matériau en dehors des vertèbres peut être critique, donnant lieu à des complications neurologiques et vasculaires pouvant entraîner la mort. Il est donc avantageux que le chirurgien puisse observer la quantité de produit injecté, sa dispersion dans la vertèbre et surtout vérifier que le matériau reste bien à l'intérieur de celle-ci.

Selon l'invention, des agents radio opaques pour l'imagerie médicale par rayons X peuvent être ajoutés dans les compositions selon l'invention. Il peut s'agir de composés iodés, ioniques ou non ioniques tels que le Ioméron^{®} ou le Iopamidol^{®}. Ces solutions iodées rendent le gel totalement radio opaque et permettent donc de le détecter par radiographie aux rayons X. Les dérivés stronsiques sont aussi envisageables comme évoqués ci avant.

Dans le cas des produits résorbables, les agents et composés radio opaques présents dans la composition, une fois injectée, présentent l'avantage supplémentaire de disparaître au cours du temps, ce qui permet d'évaluer l'efficacité de la reconstruction osseuse.

De plus, les compositions selon l'invention présentent après chauffage des propriétés rhéologiques exceptionnelles.

Ils forment des produits visqueux, de structure épaisse sous forme de pâte injectable à travers une canule à une température de l'ordre de 50°C en sortie de ladite canule.

L'invention a également pour objet l'utilisation des compositions selon l'invention en tant que substitut osseux pour vertébroplastie.

L'invention a aussi pour objet le procédé de préparation des compositions précédemment décrites. Un tel procédé comprend les étapes successives suivantes :
- préparation d'un mélange homogène comprenant le polymère thermoplastique resorbable ou non résorbable, particulièrement un polymère thermoplastique résorbable de type polycaprolactone de poids moléculaire compris entre 10000 et 100000 et optionnellement un matériau inorganique;
- Le mélange est réalisé selon un procédé mécanique ou chimique maitrisé ;
- Le mélange permet d'obtenir des formes telles qu'un bâtonnet après passage dans une presse à injecter. Alternativement le mélange peut être formulé sous la forme de granulés.

Une stérilisation du produit obtenu peut être réalisée par les moyens connus de l'homme du métier, par exemple à l'oxyde d'éthylène.

Un autre objet de la présente invention réside aussi dans un dispositif de type seringue contenant la composition selon l'invention pour l'injection en un point osseux de ladite composition afin de réaliser un comblement osseux.

En particulier, l'invention concerne donc un ensemble constitué d'une seringue et d'une dose à usage unique d'une composition selon l'invention, ladite seringue étant pré-remplie de ladite composition unitaire selon l'invention, ladite seringue permettant à la fois de stocker et d'administrer ladite composition, ladite seringue comprenant en outre un corps de seringue comportant à chacune de ses deux extrémités une ouverture, la première ouverture portant des moyens d'obturation de façon étanche, notamment un bouchon, et/ou des moyens d'administration de ladite composition, notamment une canule, la deuxième ouverture étant obturée de façon étanche par des moyens d'obturation, notamment un joint de piston, susceptibles de coulisser à l'intérieur du corps de seringue, notamment à l'aide d'une tige de piston, notamment liée auxdits moyens d'obturation susceptibles de coulisser, ledit corps de seringue, lesdits moyens d'obturation et/ou d'administration, et lesdits moyens d'obturation susceptibles de coulisser délimitant un volume dans lequel ladite composition est comprise.

Un objet de la présente invention est donc un dispositif comprenant :
- une seringue préremplie d'une dose à usage unique d'une composition pour comblement osseux, telle qu'un bâtonnet ou des granulés, ladite composition comprenant au moins un matériau polymère résorbable ou non résorbable thermoplastique, ladite composition présentant une viscosité comprise entre 40 et 4000 Pa.s à une température de 50°C environ et un module de Young compris entre 0.5 et 4 GPa à 37°C environ, ladite seringue permettant à la fois de stocker et d'administrer ladite composition, ladite seringue comprenant un corps de seringue comportant à chacune de ses deux extrémités une ouverture, la première ouverture distale portant des moyens d'obturation de façon étanche, notamment un bouchon, et/ou des moyens d'administration de ladite composition, notamment une canule, la deuxième ouverture proximale étant obturée de façon étanche par des moyens d'obturation, notamment un joint de piston, susceptibles de coulisser à l'intérieur du corps de seringue, notamment à l'aide d'une tige de piston conçue pour être déplacée en direction de l'ouverture distale, notamment liée auxdits moyens d'obturation susceptibles de coulisser, ledit corps de seringue, lesdits moyens d'obturation et/ou d'administration, et lesdits moyens d'obturation susceptibles de coulisser délimitant un volume dans lequel ladite dose est comprise ;
- un moyen de chauffage externe et structurellement séparé de la seringue et permettant son chauffage et une fusion ou un ramollissement de la dose de la composition pour comblement osseux aux fins de son administration.

Le corps de la seringue selon l'invention pourra être constitué de tout matériau résistant au chauffage à une température comprise entre 80 et 150°C, voire 120°C ; et de manière particulière le corps de la seringue est en métal. Le métal particulièrement adapté est l'aluminium compte tenu de sa légèreté et son inertie thermique permettant une bonne conductivité thermique.

La tige de piston peut être une tige filetée permettant de faire progresser la composition après chauffage et fluidification par vissage de ladite tige de piston. Dans ce cas, l'extrémité à travers laquelle passe la tige de piston pourra être dotée d'un filetage complémentaire du filetage de la tige de piston.

L'utilisation des seringues pré-remplies selon l'invention évite aux praticiens les étapes de préparation extemporanée et de transfert du produit de comblement de son contenant vers un dispositif d'administration tel qu'évoqué plus haut, ce qui, au-delà de l'amélioration du confort d'utilisation, garantit une meilleure stérilité du produit.

En effet, l'utilisation de seringue pré-remplie permet un usage unique qui d'une part facilite la manipulation et limite tous les risques de mauvais dosage.

Selon un mode de réalisation avantageux, la présente invention concerne un ensemble selon l'invention tel que décrit ci-avant, dans lequel le volume de la dose de la composition à usage unique, stockée dans la seringue est compris de 1 à 50 ml, de préférence de 2 à 35 ml, plus préférentiellement de 4 à 20 ml.

L'expression dose à usage unique s'entend dans le contexte de la présente invention en ce que la quantité de composition est adaptée pour une intervention chirurgicale sur un patient pour au moins un comblement osseux ou tout type d'intervention impliquant un complément de matière à un endroit ou de l'os fait défaut. Cette dose à usage unique s'entend aussi qu'elle est continue et sous forme à usage unique et adaptée à l'utilisation dans un dispositif selon l'invention. Cette dose peut se présenter sous la forme d'un bâtonnet de matériau polymère résorbable occupant le volume interne de la seringue mais il peut aussi s'agir de billes ou granulés dudit matériau.

Le volume de ladite dose de composition à usage unique est inférieur ou égal au volume maximal que peut contenir ladite seringue. Selon un mode de réalisation particulièrement avantageux, la présente invention concerne un ensemble tel que décrit dans lequel le volume de la dose de ladite composition à usage unique, stockée dans ladite seringue est compris de 1 à 20 ml, de préférence de 2 à 10 ml, plus préférentiellement de 4 à 10 ml. Selon un autre mode de réalisation particulièrement avantageux, la présente invention concerne un dispositif dans lequel le volume de la dose de ladite composition à usage unique, stockée dans ladite seringue est compris de 10 à 50 ml, de préférence de 10 à 35 ml, plus préférentiellement de 10 à 20 ml.

Selon un mode de réalisation particulièrement avantageux, la présente invention concerne un ensemble dans lequel le ou les moyens d'administration de la composition est ou sont aboutés à l'extrémité du corps de la seringue et comprennent ou consistent en une canule dont le diamètre est compris entre 8 et 15 gauges, en particulier de 10 à 14 gauges.

Le volume de la seringue peut être modifié en faisant coulisser lesdits moyens d'obturation à l'intérieur du corps, en direction de la première ouverture distale, ladite ouverture étant en particulier libre de moyens d'obturation. Lorsque la première ouverture distale porte des moyens d'obturation, notamment un bouchon et des moyens d'administration, notamment une canule, lesdits moyens d'obturation, dans un premier temps étanche, sont dans un deuxième temps percés lors de la mise en place desdits moyens d'administration. Dans ce cas, il n'est pas nécessaire de remplacer lesdits moyens d'obturation par lesdits moyens d'administration pour administrer ladite composition. Une tige de piston peut être adaptée au dit joint de piston, pour permettre l'administration de ladite composition, ladite administration étant manuelle, ou réalisée par des moyens d'administration automatisée.

Dans un mode de réalisation particulier, la seringue préremplie selon l'invention, avec ou sans moyen d'administration, est chauffée à une température proche de ou supérieure à la température de fusion de la composition pour comblement osseux contenue dans le corps de la seringue.

Ce chauffage peut être réalisé par tout moyen adapté et connu de l'homme du métier mais qui est externe et structurellement séparé de la seringue. Cette caractéristique du dispositif selon l'invention permet en effet de disposer d'un moyen de stockage et d'administration de la Composition, ie la seringue, dénué de systèmes électriques ou électronique de chauffage intégré comme cela existe dans l'art antérieur. Cette configuration permet de disposer d'un système simple, économique et robuste à fabriquer et à utiliser. En particulier, en préalable à son utilisation, la seringue préremplie, équipée ou non des moyens d'administration, pourra être disposée dans un four thermostaté à une température comprise entre 80°C et 150°C, particulièrement entre 80°C et 120°c, plus particulièrement entre 80 et 90°C. La seringue pourra tout aussi bien être chauffée à l'aide d'un fourreau ou collier chauffant de type dimensionné pour recevoir le corps de la seringue équipé ou non de canule d'injection.

Le chauffage permet d'approcher, d'atteindre ou dépasser la température de fusion du matériau polymère thermoplastique et de rendre la composition suffisamment malléable ou fluide pour une extrusion et une injection aisée. Selon la nature du polymère ou du mélange contenant le polymérique thermoplastique on pourra laisser le dispositif pendant un temps certain dans le système de chauffage afin de fluidifier suffisamment la composition. Une fois cet état de fluidité ou de malléabilité atteint, les inventeurs ont pu noter une hystérèse en ce, qu'en refroidissant, la viscosité est toujours inférieure - pour une température donnée - à la viscosité observée lors de la montée en température. Ceci permet ainsi d'obtenir une composition malléable, extrudable et injectable aux environs de 50°C après que celle-ci a été chauffée à 90°C ou 100°C, c'est-à-dire proche du point de fusion du polymère.

La seringue est donc utilisée comme moyen de stockage ou réservoir caloporteur.

Dans un mode particulier le moyen de chauffage externe et séparé structurellement de la seringue peut être un four convenable pour la thermostation de la seringue contenant la composition selon l'invention et se présente sous la forme d'un cylindre dont le diamètre est substantiellement égal tout en étant légèrement supérieur à celui de la seringue afin de pouvoir introduire celle-ci dans le corps dudit four. De même la longueur de ce cylindre sera substantiellement égale ou légèrement supérieur à la longueur hors tout de l'ensemble selon l'invention. L'ensemble pouvant ou non comprendre une canule aboutée à une extrémité, la longueur du cylindre du four pourra être telle qu'elle est au moins égale à la longueur de la seringue en ce compris la canule, si présente, afin d'assurer le chauffage de cette canule.

C'est aussi un objet de la présente invention que de fournir un dispositif selon l'invention dans lequel le moyen de chauffage comprend un four se présentant sous la forme d'un cylindre chauffant dont le diamètre est substantiellement égal tout en étant légèrement supérieur à celui de la seringue afin de pouvoir introduire celle-ci dans le corps dudit four et dont la longueur est substantiellement égale ou légèrement supérieure à la longueur du dispositif, avec ou sans la canule aboutée.

Le cylindre chauffant du four peut être équipé de résistances électriques chauffantes assurant l'élévation de température ou de tout autre moyen permettant cette élévation de température, par exemple l'induction.

En effet, la température de fusion du matériau polymère thermoplastique résorbable ou non résorbable de la composition pour comblement osseux selon l'invention s'établit entre 50 et 120°C, particulièrement entre 50°C et 90°C, plus particulièrement encore entre 50 et 70°C et ce selon la nature du polymère, le poids moléculaire du polymère, plus particulièrement du polymère résorbable de polycaprolactone ainsi que de la quantité de ce polymère, ou des ratio des polymères dans le cas de mélange physiques, dans ladite composition. Comme évoqué plus avant, plus le poids moléculaire est élevé, plus la température de fusion est élevée. En outre, la présence de composé inorganique de type biomatériau phosphocalcique dans la composition aura tendance à baisser la température de fusion ou de ramollissemnt de la composition comprenant le polymère thermoplastique. Cela permet ainsi d'utiliser des polymères dont la température de fusion si situe dans une gamme de l'ordre de 100°C voire au-dessus.

Ainsi, la température de thermostatation de l'ensemble selon l'invention, c'est-à-dire de la seringue contenant la dose de composition à usage unique pour comblement osseux, sera déterminée par le volume et la nature de ladite composition.

Comme évoqué plus avant, une température de thermostatation de l'ensemble selon l'invention de l'ordre de 90°C pendant un temps de l'ordre 3 à 20 minutes, par exemple, permettant d'atteindre une température de 80°C environ au cœur de la dose à usage unique de la composition permet ainsi une fluidification de ladite composition contenue dans le corps de seringue compatible avec une injection, en particulier dans le corps vertébral du patient à travers une tubulure adéquate. Cette température, à cœur, pourra bien sûr être adaptée en fonction de la nature de la composition mise en œuvre.

En effet, cette température (de l'ordre de 80°C) est certes non compatible avec les tissus mais compte tenu des transferts et déperditions de chaleur ainsi que du gradient de température au sein des pièces du dispositif au moment de l'injection, la température de la canule au contact des tissus sera de l'ordre de 50°C et la température de la composition dans l'aiguille ainsi qu'au site d'injection (par exemple la vertèbre) sera aussi de cet ordre de grandeur ce qui évitera de bruler les tissus.

En outre, à cette température (de l'ordre de 50 à 60°C) au sein de la composition comprise dans l'aiguille, ladite composition est fluide et l'aiguille peut ainsi être ôtée sans difficulté.

En effet, comme indiqué précédemment, la première extrémité du corps de la seringue est équipée de moyens de distribution de la composition, en particulier une canule chirurgicale.

La longueur de cette canule pourra être choisie en fonction de l'application désirée et sera de l'ordre de 100 à 150 mm.

Typiquement, lors de l'extrusion de la composition fluidifiée via l'application d'une force au niveau de la tige de piston, la progression de la composition fluide le long de la canule implantée dans le corps du patient entraîne un refroidissement de celle-ci. De même une fois que la composition arrive dans la cavité à combler, une baisse de température s'observe aussi.

Il convient donc de prévoir une température de la composition au sein du corps de la seringue adéquate afin qu'après la progression le long d'une canule disposée dans une cavité osseuse du patient, via un trocart, et au débouché de ladite cavité, la composition ne se soit pas solidifiée. Ainsi, une température de la composition préférée selon la présente invention, dans le corps de la seringue de l'ordre de 80°C, permet d'obtenir une composition fluide et injectable et qui conserve sa fluidité une fois arrivée à l'extrémité d'une canule de 150 mm en présentant une température de 45 à 50°C.

Au débouché dans la cavité osseuse, au contact du milieu intérieur du patient, la température de la composition s'établit aux environs de 40 à 45°C environ ce qui permet à ladite composition encore plus ou moins fluide de combler efficacement le vide.

Un tel profil de température permet de concilier une délivrance et une injection efficace et de préserver les tissus du patient de tout risque de nécrose.

Un objet de l'invention concerne aussi un kit comprenant un ensemble selon l'invention et une canule pour injection de la composition.

Au moment de la mise en œuvre de l'ensemble ou du kit selon l'invention, l'obturateur de l'extrémité de la seringue est ôté, une canule, particulièrement celle comprise dans le kit, est fixée à ladite extrémité et l'ensemble est mis en chauffe dans un dispositif de chauffage adéquat tel qu'un four cylindrique comme évoqué plus avant par exemple.

Dans le cas de l'utilisation de la composition en vertébroplastie, pendant le chauffage de l'ensemble, le chirurgien va pouvoir procéder au positionnement d'un trocart au niveau de la vertèbre fracturée ou à traiter par exemple. Cela peut être réalisé en toute sécurité sous contrôle radiographique continu pendant l'opération. Un ballonnet peut alors être inséré à travers le trocart dans la vertèbre, puis gonflé. On peut ainsi restaurer la vertèbre, qui est soulevée, permettant la formation d'une cavité. Après le retrait du ballonnet, la canule de la seringue est introduite dans le trocart et la composition est extrudée et on remplit la cavité de la composition de comblement.

La composition est entièrement dure après quelques minutes ou dizaines de minutes selon la nature et la quantité de polymère thermoplastique.

Le corps de la seringue est préférentiellement en métal, et plus particulièrement en aluminium ce qui permet un transfert rapide de la chaleur vers l'intérieur et permet une liquéfaction de la composition contenue tout en garantissant une inertie thermique suffisante pour conserver la composition dans un état fondu et à une viscosité satisfaisante pour être extrudée.

En outre, si le chirurgien souhaite, en cours d'administration, rendre plus fluide la composition contenue dans la seringue et/ou la canule, il suffira de la réintroduire dans le moyen de chauffage pour assurer une liquéfaction de ladite composition aux fins de sa délivrance.

Dans un mode de réalisation particulier, et ce afin de faciliter la manipulation de la seringue par le chirurgien, un fourreau isolant enveloppant entièrement ou partiellement la seringue et les moyens d'obturation et/ou d'administration de la composition est aménagé au niveau du corps de ladite seringue.

Ce fourreau peut être solidaire du corps de la seringue ou amovible.

Ce fourreau, solidaire ou amovible, peut coulisser selon l'axe longitudinal du corps de la seringue et recouvrir totalement ou partiellement ledit corps, se verrouiller et permettre un maintien du corps de la seringue tout en laissant progresser la tige de piston, par exemple par vissage ou simple poussée. Un tel fourreau permet de protéger le chirurgien ou toute personne manipulant la seringue d'une brulure du fait de la température du corps de la seringue au sortir du dispositif de chauffage. Le fourreau peut aussi par exemple se présenter sous la forme de deux demi coques reliées par une charnière prenant en tenaille la seringue et assurant son maintien.

En effet, au moment où l'ensemble est introduit dans un dispositif de chauffage, le fourreau isolant est coulissé, ou ôté, de manière à ce qu'il ne recouvre plus le corps de la seringue qui est ainsi chauffé indépendamment dudit fourreau. Une fois le corps de la seringue porté à la température désirée, ledit fourreau est coulissé en sens inverse, de manière à recouvrir le corps de la seringue. En cas de fourreau amovible, celui-ci est repositionné sur le corps de seringue. Ce fourreau peut être verrouillé dans une ou l'autre ou les deux positions de coulissement, préférentielle dans la position recouvrant totalement le corps de la seringue afin de permettre une préhension dudit corps et une poussée de la composition contenue. Le verrouillage peut être réalisé via un système de baïonnette via un mouvement de poussé-tourné par exemple.

Le fourreau protecteur isolant peut être constitué de tout matériau pour peu qu'il soit faiblement conducteur de chaleur voire isolant thermique. Il peut s'agir par exemple de résine de silicone ou de polymère polycarbonate ou tout autre matériau à faible conductivité thermique.

La présente invention permet de s'affranchir des étapes de préparation extemporanée des ciments ou des manipulations des matériaux sous forme de granules qui sont introduits dans une chambre de chauffe d'un pistolet chauffant.

L'ensemble selon l'invention, une seringue pré remplie, peut être stérilisée et emballée et pourra être utilisée directement au bloc opératoire en minimisant les manipulations.

En outre, après chauffage et lors de la manipulation, si la composition contenue dans le corps de la seringue ou déjà présente dans la canule venait à solidifier, il suffit de remettre l'ensemble à chauffer dans le moyen de chauffage externe, tel que four ou fourreau chauffant, pour fluidifier à nouveau la composition et reprendre l'injection.

Cet avantage n'est pas possible avec les dispositifs de type pistolet chauffant existants car la canule qui est associée n'est pas chauffée et lorsque la composition qui y est contenue est solidifiée, il faut ôter cette canule et recommencer le chauffage.

Le système selon l'invention présente l'avantage de comprendre une seringue structurellement séparée par rapport à tout moyen de chaleur ou d'énergie extérieure une fois chauffé à la température adéquate ce qui en fait un système caloporteur autonome. Ainsi l'absence de batterie, de résistance électrique, de fils électriques assurant la connexion à une source d'électricité extérieur permet une manipulation aisée pour le chirurgien et permet d'éliminer tout risque électrique pour le patient. Enfin, la conception mécaniquement simple du système selon l'invention permet sa fabrication en grande série et ce pour un prix compétitif.

Un système selon l'invention présente donc l'avantage d'être facile à manipuler, facile et peu onéreux à fabriquer et peut être jeté après usage.

### EXEMPLE 1

### Mesure des propriétés rhéologiques

Le matériel utilisé consiste en:
- Viscosimètre MCR51
- Enceinte climatique 3-EC-02 (SLH 100 - Sécasi)
- Logiciel Rheoplus

Les essais pour la mesure de la viscosité du polymère polycaprolactone sont réalisés à l'aide d'un rhéomètre/viscosimètre Anton Paar Modèle MCR51 en mode de cisaillement dynamique (1Hz). Cet appareil permet d'enregistrer l'évolution de la viscosité complexe en fonction du temps et à une température constante. Afin de se rapprocher des conditions réelles d'utilisation de l'injecteur chauffant, le polymère est fondu à 90°C dans l'appareil puis une température de consigne est fixée (température à laquelle le matériau arrivera dans le site osseux à combler). L'acquisition des mesures est lancée lorsque la température désirée (50°C) du support est stable.

### EXEMPLE 2.

Composition PCL + agent phosphocalcique stronsique
PCL avec masse moléculaire Mn = 30 000g/mol + β-TCP strontium
Ratio PCL/agent phosphocalcique
PCL/β-TCP(Sr) = 70/30
Granulométrie agent phosphocalcique
β-TCP(Sr)= 30 µm
Dispositif/Seringue d'injection

Dispositif d'injection selon l'invention avec une cartouche métallique chargée d'un bâtonnet de matière PCL/TCP(Sr) connectée à une canule de gauge 11 pouvant s'introduire dans un trocard de gauge 10. La chauffe du dispositif avant le transfert pour l'injection dans la vertèbre est réalisée dans une étuve thermostatée à 90°C.

## Revendications

1. Dispositif comprenant :
- une seringue préremplie d'une dose à usage unique d'une composition pour comblement osseux, telle qu'un bâtonnet ou des granulés, ladite composition comprenant au moins un matériau polymère thermoplastique résorbable ou non résorbable, ladite composition présentant une viscosité comprise entre 40 et 4000 Pa.s à une température de 50°C environ et un module de Young compris entre 0.5 et 4 GPa à 37°C environ, ladite seringue permettant à la fois de stocker et d'administrer ladite composition, ladite seringue comprenant un corps de seringue comportant à chacune de ses deux extrémités une ouverture, la première ouverture distale portant des moyens d'obturation de façon étanche, notamment un bouchon, et/ou des moyens d'administration de ladite composition, notamment une canule, la deuxième ouverture proximale étant obturée de façon étanche par des moyens d'obturation, notamment un joint de piston, susceptibles de coulisser à l'intérieur du corps de seringue, notamment à l'aide d'une tige de piston conçue pour être déplacée en direction de l'ouverture distale, notamment liée auxdits moyens d'obturation susceptibles de coulisser, ledit corps de seringue, lesdits moyens d'obturation et/ou d'administration, et lesdits moyens d'obturation susceptibles de coulisser délimitant un volume dans lequel ladite dose est comprise ;
- un moyen de chauffage externe et structurellement séparé de la seringue et permettant son chauffage et une fusion ou un ramollissement de la dose de la composition pour comblement osseux aux fins de son administration.

2. Dispositif selon la revendication 1, **caractérisé en ce que** le corps de la seringue est en métal.

3. Dispositif selon la revendication 1, **caractérisé en ce que** le volume de la dose de la composition à usage unique, stockée dans la seringue est compris de 1 à 50 ml.

4. Dispositif selon la revendication 1, **caractérisé en ce que** les moyens d'administration de la composition sont aboutés à l'extrémité du corps de la seringue et comprennent une canule dont le diamètre est compris entre 8 et 15 gauges, en particulier de 10 à 14 gauges.

5. Dispositif selon la revendication 1, **caractérisé en ce que** le matériau polymère est un polymère résorbable et est choisi dans le groupe constitué par l'acide poly(lactique-co-glycolique), l'acide polylactique, l'acide polyglycolic, le poly(caprolactone), le polyhydroxybutyrate, le polyglycerolsebacate, le poly(dioxanone) utilisés seuls ou en mélange.

6. Dispositif selon la revendication 1, **caractérisé en ce que** le matériau polymère est un polymère non résorbable et est choisi dans le groupe constitué par les polymères et copolymères acrylates et méthacrylate, polyuréthane, polyéthylène, polypropylène, utilisés seuls ou en mélange.

7. Dispositif selon la revendication 1, **caractérisé en ce que** le matériau polymère est un polycaprolactone de poids moléculaire compris entre 30000 et 60000.

8. Dispositif selon la revendication 1, **caractérisé en ce que** la composition comprend en outre un matériau inorganique, plus particulièrement un biomatériau inorganique, plus particulièrement encore un biomatériau phosphocalcique.

9. Dispositif selon la revendication 8, **caractérisé en ce que** le biomatériau phosphocalcique est une biocéramique phosphocalcique, particulièrement une biocéramique phosphocalcique strontique 1 .

10. Dispositif selon la revendication 9, caractérisé en que la biocéramique comprend ou consiste en un ou plusieurs composés phosphocalciques frittés choisis dans le groupe consistant en hydroxyapatite (HA), alpha- et beta-tricalcium phosphate (α-TCP, β-TCP) et matériau phosphocalcique biphasé (BCP) utilisés seuls ou en mélange.

11. Dispositif selon la revendication 1, **caractérisé en ce que** le ratio massique polymère/matériau inorganique de la composition est compris entre 100/0 et 40/60, plus particulièrement entre 90/10 et 50/50, plus particulièrement encore entre 80/20 et 70/30.

12. Dispositif selon la revendication 1 **caractérisé en ce que** la composition se présente sous forme de bâtonnet ou granulés et comprend en poids 40 à 100 % d'un polymère de polycaprolactone de poids moléculaire compris entre 30000 et 60000 , 0 à 60 % d'un biomatériau phosphocalcique, en particulier un biomatériau phosphocalcique comprenant de l'hydroxyapatite et/ou du phosphate tricalcique.

13. Dispositif selon la revendication 1 **caractérisé en ce qu'**un fourreau isolant enveloppant entièrement ou partiellement la seringue et les moyens d'obturation et/ou d'administration de la composition est aménagé au niveau du corps de ladite seringue.

14. Dispositif selon l'une des revendications précédentes **caractérisé en ce que** le moyen de chauffage comprend un four convenable pour la thermostation dudit dispositif, ledit four se présentant sous la forme d'un cylindre chauffant dont le diamètre est substantiellement égal tout en étant légèrement supérieur à celui de la seringue afin de pouvoir introduire celle-ci dans le corps dudit four et dont la longueur est substantiellement égale ou légèrement supérieure à la longueur hors tout du dispositif.

## Patentansprüche

1. Vorrichtung, umfassend:
- eine Spritze, die mit einer Einwegdosis einer Zusammensetzung zum Auffüllen von Knochen, wie einem Stäbchen oder Granulat, gefüllt ist, wobei die Zusammensetzung wenigstens ein resorbierbares oder nicht resorbierbares thermoplastisches Polymermaterial umfasst, wobei die Zusammensetzung eine Viskosität zwischen 40 und 4000 Pa.s bei einer Temperatur von etwa 50 °C und ein Young'sches Modul zwischen 0,5 und 4 GPa bei etwa 37 °C aufweist, wobei die Spritze sowohl die Aufbewahrung als auch die Verabreichung der Zusammensetzung ermöglicht, wobei die Spritze einen Spritzenkörper umfasst, der an jedem seiner beiden Enden eine Öffnung aufweist, wobei die erste distale Öffnung Mittel zum dichten Verschließen umfasst, insbesondere einen Stopfen, und/oder Mittel zur Verabreichung der Zusammensetzung, insbesondere eine Kanüle, wobei die zweite proximale Öffnung durch Verschlussmittel dicht verschlossen ist, insbesondere durch eine Kolbendichtung, die im Inneren des Spritzenkörpers gleiten können, insbesondere mit Hilfe einer Kolbenstange, die dazu ausgebildet ist, in Richtung der distalen Öffnung verschoben zu werden, insbesondere mit den gleitfähigen Verschlussmitteln verbunden, wobei der Spritzenkörper, die Verschluss- und/oder Verabreichungsmittel und die gleitfähigen Verschlussmittel ein Volumen begrenzen, in dem die Dosis enthalten ist;
- ein externes und strukturell von der Spritze getrenntes Heizmittel, das deren Erhitzung und ein Schmelzen oder Erweichen der Dosis der Knochenfüllstoffzusammensetzung zum Zweck ihrer Verabreichung ermöglicht.

2. Vorrichtung nach Anspruch 1, **dadurch gekennzeichnet, dass** der Spritzenkörper aus Metall besteht.

3. Vorrichtung nach Anspruch 1, **dadurch gekennzeichnet, dass** das Volumen der Dosis der Zusammensetzung zur einmaligen Verwendung, die in der Spritze aufbewahrt wird, zwischen 1 und 50 ml beträgt.

4. Vorrichtung nach Anspruch 1, **dadurch gekennzeichnet, dass** die Mittel zur Verabreichung der Zusammensetzung an das Ende des Spritzenkörpers angefügt sind und eine Kanüle mit einem Durchmesser von 8 bis 15 Gauge, insbesondere 10 bis 14 Gauge, umfassen.

5. Vorrichtung nach Anspruch 1, **dadurch gekennzeichnet, dass** das Polymermaterial ein resorbierbares Polymer ist und gewählt ist aus der Gruppe, umfassend Poly(milch-co-glykolsäure), Polymilchsäure, Polyglykolsäure, Poly(caprolacton) , Polyhydroxybutyrat, Polyglycerolsebacat, Poly(dioxanon), die allein oder in Mischungen verwendet werden.

6. Vorrichtung nach Anspruch 1, **dadurch gekennzeichnet, dass** das Polymermaterial ein nicht resorbierbares Polymer ist und aus der Gruppe gewählt ist, umfassend Polymere und Copolymere von Acrylaten und Methacrylat, Polyurethan, Polyethylen, Polypropylen, die allein oder in Mischungen verwendet werden.

7. Vorrichtung nach Anspruch 1, **dadurch gekennzeichnet, dass** das Polymermaterial ein Polycaprolacton mit einem Molekulargewicht zwischen 30000 und 60000 ist.

8. Vorrichtung nach Anspruch 1, **dadurch gekennzeichnet, dass** die Zusammensetzung ferner ein anorganisches Material umfasst, bevorzugt ein anorganisches Biomaterial, stärker bevorzugt ein Phosphorkalzium-Biomaterial.

9. Vorrichtung nach Anspruch 8, **dadurch gekennzeichnet, dass** das Phosphorkalzium-Biomaterial eine Phosphorkalzium-Biokeramik ist, insbesondere eine strontische Phosphorkalzium-Biokeramik.

10. Vorrichtung nach Anspruch 9, **dadurch gekennzeichnet, dass** die Biokeramik eine oder mehrere gesinterte Phosphorkalziumverbindungen umfasst oder daraus besteht, gewählt aus der Gruppe, umfassend Hydroxylapatit (HA) , alpha- und beta-Trikalziumphosphat (α-TCP, β-TCP) und zweiphasiges Phosphorkalziummaterial (BCP), die allein oder in Mischungen verwendet werden.

11. Vorrichtung nach Anspruch 1, **dadurch gekennzeichnet, dass** das Massenverhältnis Polymer/anorganisches Material in der Zusammensetzung zwischen 100/0 und 40/60, bevorzugt zwischen 90/10 und 50/50, noch stärker bevorzugt zwischen 80/20 und 70/30 liegt.

12. Vorrichtung nach Anspruch 1, **dadurch gekennzeichnet, dass** die Zusammensetzung in Form von Stäbchen oder Granulat vorliegt und 40 bis 100 Gew.-% eines Polycaprolactonpolymers mit einem Molekulargewicht zwischen 30000 und 60000 und 0 bis 60 Gew.-% eines Phosphorkalzium-Biomaterials, insbesondere eines Phosphorkalzium-Biomaterials, umfassend Hydroxylapatit und/oder Trikalziumphosphat, umfasst.

13. Vorrichtung nach Anspruch 1, **dadurch gekennzeichnet, dass** eine isolierende Hülle, die die Spritze und die Mittel zum Verschließen und/oder zur Verabreichung der Zusammensetzung ganz oder teilweise umhüllt, am Körper der Spritze angebracht ist.

14. Vorrichtung nach einem der vorhergehenden Ansprüche, **dadurch gekennzeichnet, dass** das Heizmittel einen Ofen umfasst, der für die Thermostatisierung der Vorrichtung geeignet ist, wobei der Ofen die Form eines Heizzylinders hat, dessen Durchmesser im Wesentlichen gleich oder etwas größer als der der Spritze ist, um diese in den Körper des Ofens einführen zu können, und dessen Länge im Wesentlichen gleich oder etwas größer als die Gesamtlänge der Vorrichtung ist.

## Claims

1. Device comprising:
- a syringe prefilled with a single-use dose of a composition for bone filling, such as a rod or pellets, said composition comprising at least one resorbable or non-resorbable thermoplastic polymer material, said composition having a viscosity between 40 and 4000 Pa.s at a temperature of about 50°C and a Young's modulus between 0.5 and 4 GPa at about 37°C, said syringe enabling both the storage and administration of said composition, said syringe comprising a syringe body having an opening at each of its two ends, the distal first opening bearing sealing means for sealing it closed, in particular a plug, and/or means for the administration of said composition, in particular a cannula, the proximal second opening being sealed closed by sealing means, in particular a piston seal, slidable inside the syringe body, in particular by means of a piston rod adapted to be moved toward the distal opening, in particular connected to said slidable sealing means, said syringe body, said sealing and/or administration means, and said slidable sealing means defining a volume within which said dose is contained;
- an external heating means structurally separate from the syringe and making it possible to heat it and melt or soften the dose of the composition for bone filling for the purposes of its administration.

2. Device according to claim 1, wherein the syringe body is made of metal.

3. Device according to claim 1, wherein the volume of the single-use dose of the composition stored in the syringe is between 1 and 50 ml.

4. Device according to claim 1, wherein the means for administration of the composition are joined to the end of the syringe body and comprise a cannula the diameter of which is between 8 gauge and 15 gauge, in particular 10 gauge to 14 gauge.

5. Device according to claim 1, wherein the thermoplastic polymer material is a resorbable polymer and is selected from the group consisting of poly(lactic-co-glycolic acid), polylactic acid, polyglycolic acid, polycaprolactone, polyhydroxybutyrate, poly(glycerol sebacate), poly(dioxanone), alone or in admixture.

6. Device according to claim 1, wherein the thermoplastic polymer material is a non-resorbable polymer and is selected from the group consisting of polymers and copolymers of acrylates and methacrylate, polyurethane, polyethylene, alone or in admixture.

7. Device according to claim 1, wherein the thermoplastic polymeric material is a polycaprolactone having a molecular weight between 30,000 and 60,000.

8. Device according to claim 1, wherein the composition further comprises an inorganic material, particularly an inorganic biomaterial, more particularly a calcium phosphate biomaterial.

9. Device according to claim 8, wherein the calcium phosphate biomaterial is a calcium phosphate bioceramic, particularly a strontium-containing calcium phosphate bioceramic.

10. Device according to claim 9, wherein the bioceramic comprises or consists of one or more sintered calcium phosphate compounds selected from the group consisting of hydroxyapatite (HA), alpha- and beta-tricalcium phosphate (α-TCP, β-TCP), and biphasic calcium phosphate material (BCP), alone or in admixture.

11. Device according to claim 1, wherein the weight ratio of polymer/inorganic material of the composition is between 100/0 and 40/60, particularly between 90/10 and 50/50, more particularly between 80/20 and 70/30.

12. Device according to claim 1, wherein the composition is in the form of a rod or pellets and comprises by weight 40 to 100% of a polycaprolactone polymer having a molecular weight between 30,000 and 60,000, 0 to 60% of a calcium phosphate biomaterial, in particular a calcium phosphate biomaterial comprising hydroxyapatite and/or tricalcium phosphate.

13. Device according to claim 1, wherein an insulating sheath completely or partially surrounding the syringe and the composition administration and/or sealing means is arranged on the syringe body.

14. Device according to one of the preceding claims, wherein the heating means comprises an oven suitable for regulating the temperature of said device, said oven in the form of a heating cylinder having a diameter substantially equal to but slightly greater than that of the syringe in order to allow insertion of the syringe into the body of the oven and having a length substantially equal to or slightly greater than the length overall of the device.
